# EUROPEAN PATENT SPECIFICATION

(11) **EP 1 254 663 B1**
(45) Date of publication and mention of the grant of the patent: **10.11.2004**
(21) Application number: 01110873.5
(22) Date of filing: 04.05.2001
(51) Int. Cl.: A61K 31/70, C07K 14/34

(54) **Anti-virus agent**
Anti-virales Reagens
Agent anti-virale

(43) Date of publication of application: 06.11.2002
(73) Proprietor: Technologie Integrale Ltd., RAMSEY, Isle of Man IM8 IRD (GB)
(72) Inventor: Papuashvili, Marina N., Moskow (RU)
(74) Representative: Becker Kurig Straus

(56) References cited:
- EP-A- 0 479 180
- WO-A-90/07986
- HARRISON G S ET AL: "ACTIVATION OF A DIPHTERIA TOXIN A GENE BY EXPRESSION OF HUMAN IMMUNODEFICIENCY VIRUS-1 THAT AND REV PROTEINS IN TRANSFECTED CELLS" HUMAN GENE THERAPY, XX, XX, vol. 2, no. 1, 1991, pages 53-60, XP000651800 ISSN: 1043-0342
- PALESE P ET AL: "NEGATIVE-STRAND RNA VIRUSES: GENETIC ENGINEERING AND APPLICATIONS" PROCEEDINGS OF THE NATIONAL ACADEMY OF SCIENCES OF USA, NATIONAL ACADEMY OF SCIENCE. WASHINGTON, US, vol. 93, 1 October 1996 (1996-10-01), pages 11350-11354, XP000196755 ISSN: 0027-8424

### SEQUENCE LISTING

<110> Technologie Intègrale
<120> Anti-RNA Virus Composition
<130> 50553
<140>
   <141>
<160> 2
<170> PatentIn Ver. 2.1
<210> 1
   <211> 39
   <212> DNA
   <213> Hepatitis C Virus
<400> 1
   taatacgatt cactataggg aattccacag ctgggcgga                          39
<210> 2
   <211> 19
   <212> DNA
   <213> Hepatitis C Virus
<400> 2
   ttcacgcaga aagcgtcta                                                 19

## Description

The present invention pertains to an anti-virus agent acting against single stranded RNA (+) viruses. In particular, the present invention relates to an anti-virus agent containing a construct comprising a nucleotide sequence capable to direct the synthesis of the complementary strand and a gene encoding a toxin operably linked to a regulon, wherein the gene encoding the toxin is invertedly oriented in the construct. The present invention also pertains to the use of said anti-virus agent for preparing a medicament for treating viral diseases.

Single stranded RNA (+) viruses are positive-strand viruses with no DNA stage during propagation in a host cell. The viruses of that group multiply their genomic RNA by means of enzymes encoded by the virus itself, generically designated RNA-dependent RNA-polymerase (RdRp). In most cases said RdRp a complex of so called non-structural (NS) proteins, which do not form part of the virion.

This enzyme complex of NS-proteins recognizes untranslated sequences/regions of the viral RNA (termed UTR), which are generally located at the 5' and/or the 3' end of the viral genome, each, and effects the synthesis of the complementary strand, i.e. the (-) strand) of the single stranded (+) RNA (Ekland et al., Nature **382** (1986), 373-376). In addition, this complex also accounts for the synthesis of multiple copies of the genomic viral RNA in response to the double stranded RNA, the duplex of the sense (+) and antisense (-) RNA-strand formed (Bartenschlager et al., J. Gen. Vir. **71** (2000), 1497-1505).

One of the most prominent members of such ss(+)RNA-viruses is the hepatitis virus several subgroups of which have been found until now. Hepatitis C virus (HCV), the most common type, is a positive-stranded, enveloped virus which is evolutionarily related to the flaviviruses and pestiviruses. The HCV genome consists of a 5'-untranslated region (5'-UTR), a long open reading frame (ORF) of between 9030 and 9099 nucleotides and a 3'-UTR. The 3' end of the genome has been reported to contain a short stretch of poly(A) in type I strains, which finding has, however, not yet been confirmed in type II-, III- or IV-strains. Sequences in the 5'-UTR have been reported to exhibit negative translational control over viral gene expression.

The RdRp-complex of HCV has been found to interact with a region of between 27 and 45 nucleotides in the 3' UTR which functions as a structural clue for the initiation of the complementary strand (the (-) strand) and eventually viral genomic replication.

The hepatitis C virus group is responsible for most cases of non-A, non-B hepatitis. Although non-A, non-B hepatitis may be caused by many different viruses, the principal cause is HCV. The infection is transmitted through blood transfusion, percutaneous infusion from illicit drug usage, renal organ transplant, and maternal-neonatal transfer. Up to 20% of sporadic outbreaks of hepatitis are caused by HCV.

The association of hepatitis C virus in posttransfusion hepatitis (>95% of the cases) and the subsequent observation that infection with this virus, more so than with hepatitis B virus, correlates strongly with the development of hepatocellular carcinoma and liver cirrhosis has led to an intense study of the molecular biology of the viral genes.

So far, there is no potent medication available for a treatment of viral diseases brought about by ss(+)RNA viruses, such as the e.g. the foot and mouth disease or the Dengue fever. For hepatitis brought about by HCV a known therapy involves the administration of drugs containing IFN-α and/or IFN-γ. However, the administration of the drugs on their own did not prove to be very effective and also a combined application of both substances did not produce the desired results (Samuel et al., Virology **130** (1983), 474-484).

Therefore, there exists a strong need in the art for a substantive remedy for treating viral diseases brought about by ss(+)RNA-viruses, such as the HCV.

Hence, an object of the present invention resides in providing such means for treating viral diseases brought about by ss(+)RNA-viruses.

This problem has been solved by providing an anti-virus agent, directed against single stranded (+) RNA viruses, comprising the following components (A) and (B), wherein component (A) is a nucleotide sequence directing the synthesis of the complementary strand of the single stranded (+) RNA virus, and wherein component (B) is a nucleotide sequence containing at least a regulatory region operably linked to a structural gene encoding a toxin, with the nucleotide sequence encoding the toxin being oriented in an inverted direction with respect to the (+)-strand (sense-strand).

According to another preferred embodiment component (A) is derived from the 5'- or 3'-untranslated region of a ss(+)RNA virus, such as the hepatitis virus type B, C, D and or E, the Dengue virus, unclassified flaviviridae, Rubella virus, Yellow fever virus, bovine viral diarrhoea virus, swine fever virus, footh and mouth disease virus. According to yet another preferred embodiment said component (A) is derived from the 3'-UTR of the HCV or from a part thereof.

According to another preferred embodiment the regulatory region comprises a Shine-Dalgarno sequence or the internal ribosomal binding site (IRBS) of the genomic RNA of the poliovirus vaccine strain Sabin 2.

The toxin may be any toxin that exhibits a LD50 for vertebrates of not more than 100 ng/kg body weight and that is accepted for use in individuals, such as humans or animals or even plants (cf. CDC Final Rules). According to a preferred embodiment the toxin is selected from the group comprising the diphtheria exotoxin, the A-subunit of the diphtheria exotoxin, the Shigella toxin or the Disenteria neurotoxin.

The anti-virus agent according to the present invention may be used for the manufacture of a medicament for treating a viral disease, which disease may by caused by a hepatitis virus type A, B, C, D and or E, the Dengue virus, unclassified flaviviridae, Rubella virus, Yellow fever virus, Dengue virus, bovine viral diarrhoea virus, swine fever virus, footh and mouth disease virus.

In the figures,
Fig. 1 schematically shows the principle of constructing a genetic anti-virus; and
Fig. 2 schematically shows the structure of a GAPr.

During the extensive studies leading to the invention the present inventor has designed a novel concept for treating viral diseases brought about by ss(+)RNA-viruses, which has been termed the Genetic Antiviral Programm (GAPr).

In this respect the present invention provides a construct that comprises two main components (A) and (B). Component (A) is a nucleotide sequence that brings about the synthesis of a second, strand, which is complementary to the sense strand of the construct. Since in most of the ss(+)RNA-viruses known so far these sequences are located in the 5'-and/or 3'-untranslated regions of the virus, these sequences or functional parts thereof are the preferred sequences to be incorporated into the construct. It will be appreciated that the nucleotide sequences may be derived from the virus to be treated, but that also other nucleotide sequences may be applied as long as they enable the synthesis of the (-) strand by the polymerase complex of the virus to be treated. This nucleotide sequence is most preferably derived from the ss(+)RNA virus to be treated, since such a construct safely ensures a synthesis of the (-) strand only in the presence of the virus itself.

In the case of the HCV this component (A) is preferably the 3'-UTR or the nucleotide stretch of between 27 and 45 nucleotides in the 3'-UTR which is known to function as a structural clue for the initiation of the complementary (-)-strand.

Component (B) contains two constituents, a regulatory region and a nucleotide sequence encoding a toxin.

The regulatory region is designed to initiate the translation of the gene encoding the toxin and is therefore operably linked thereto. Such regulon may be any nucleotide sequence known to effect such initiation of translation and is preferably a regulon derived from the individual to be treated, such as the Shine Dalgarno sequence or the toxin used. In the latter case the regulon constitutes an integral part of the nucleotide encoding the toxin. However, translation may also be effected by means of viral ribosomal binding sites, such as the internal ribosomal binding site (IRBS) of the genomic RNA of the poliovirus vaccine strain Sabin 2. According to a preferred embodiment the regulon contains a translation initiation sequence used by the virus to be treated itself, so as to safeguard an unwanted translation of the polypeptide in cells that are not infected by the virus.

The second constituent of component (B) is a nucleotide sequence encoding a toxin. Yet, on the construct this nucleotide sequence is invertedly oriented, i.e. inverse to the 5' → 3' direction of the (+)-strand (the sense strand) of the construct, so that upon an unwanted formation of a RNA molecule from the construct in a host cell, again the sense strand of the construct is formed (with the toxin being arranged in the inverse direction), which does not give rise to a gene product, that is a toxin polypeptide.

However, when the construct enters a cell, which harbors a ss(+)RNA virus, that is enters an infected cell, component (A) on the construct will ensure, together with the RdRp complex provided by the virus in the cell, that the (+) strand of the construct will be transcribed into a (-)RNA strand, which the will also multiply within the cell. On this (-)RNA strand of the construct now formed the nucleotide encoding a toxin is in a direction, enabling its translation into a polypeptide, with the effect that the toxin formed in the cell will eventually kill the cell. Since the formation of the (-) RNA strand of the construct will only occur in cells infected by a virus, i.e. in cells that contain the RdRp-complex, the anti-virus agent of the present invention provides for a selective killing of infected cells.

In order to provide a proper formation of the (-) RNA strand of the construct, said component (A) needs to be arranged such, that the (-) strand, and likewise the multiple copies thereof synthesized in the cell, will contain both the regulon and the nucleotide sequence encoding the toxin. E.g. component (A) may be located upstream of the component (B), but downstream of the regulon. Likewise, component (A) may be located downstream component (B), yet in a distance, that the RNA molecule formed by means of said component (A) will still be part thereof. This may be achieved, e.g. by locating component (A) adjacent to component (B) or by modifying the nucleotide sequence downstream of component (B) such that a long transcript which will include component (A) will be formed.

The toxin may be any toxin that is capable to kill the cell, in which it is produced. According to common standards such toxins shall exhibit a LD50 for vertebrates of not more than 100 ng/kg body weight and shall be accepted for use in individuals, such as humans or animals or even plants (cf. CDC Final Rules). Examples for preferred toxins are the diphtheria exotoxin, the A-subunit of the diphtheria exotoxin, the Shigella toxin or the Disenteria neurotoxin.

The construct as such may be any DNA- or RNA-sequence, such as plasmid vectors or viral vectors. Examples for these vectors are e.g. pCI (Promega), pCMV-β, pCDM8, pcDNA 1.1 (Invitrogene) etc..

To bring the construct into a host cell the construct is brought in a suitable galenic form and administered to the patient. Such galenic forms include solutions for injections or subcutan applications, nasal instillation, peroral drug forms, encapsuation in liposomes, targeted micro-encapsulated preparates, or inclusion in a viral genome

In order to provide an improved stability of the construct versus enzymatic or chemical degradation the nucleotides may also be present in a modified form, which the skilled person may provide based on his own technical skill. Examples for such stabilization means are capping, methylation or using analogs to the common nucleotides.

As mentioned above, when the construct enters a cell in the body of the individual to be treated, the nucleotide sequence downstream of the promotor may be transcribed by means of cellular enzymes into a RNA molecule that harbors the toxin in antisense direction (component (B)) and component (A)), Yet, since said RNA molecule is subject to normal degradative processes in the cell it will disappear with the time. However, in case a virus (to be treated) is present in the said cell, this virus will provide for the RdRp-complex capable to effect synthesis of the (-)-strand of the (+) strand of the construct, or of any RNA molecule comprising said component (A),

Hence, in the latter case a RNA molecule will be formed that now contains the nucleotide sequence encoding the toxin in a correct reading direction and consequently the toxin will be synthesized in the cell, eventually killing the cell.

In consequence, the present invention provides for a selective killing of cells infected by a virus (to be treated), since only in those cells the "antisense-toxin" will be transcribed in the sense direction for its translation into a polypeptide. In virus-free cells no formation of the (-) -strand comprising the gene encoding the toxin in sense-direction is present, so that no damage to uninfected cells will occur.

Without wishing to be bound by any theory it is presently also believed that in the process of dying, parts of the infected cell may become part of the virions, which includes the incorporation one or more copies of of the multiplied construct into the virion and its transfer to other cells to be infected. Hence, the present construct may not only selectively kill cells infected by the viruses but also provides a transfer to additional cells that are infected.

Therefore, the present invention provides the following advantages. First, a selective killing of cells infected with ss(+)RNA-containing viruses takes place. Second, the construct can be inserted into the cell either as DNA, when it is part of a vector plasmid, or as RNA, when it is introduced after its transcription, and also as a result of an earlier multiplication. Third, the construct is not capable of forming the toxin without the viral enzymatic apparatus which makes the present system safe. Fourth, the construct is capable of multiplication within the host cell, so that minimal doses may be administered to the individual.

The following examples illustrate the invention without limiting it thereto.

### Example 1

### Construction of the vector

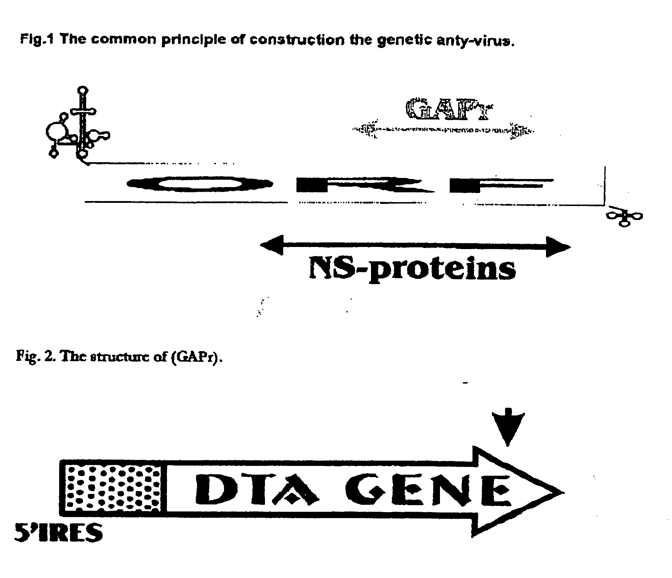

The GAPr structure as shown in Fig. 2 and limited by the sites for restriction endonucleases Not I and Sif I, is comprised of 2 parts. The regulatory part is represented by the IRES sequence of the genomic RNA of the vaccine-strain Sabin 2 polio virus (PV), its structural part, by the ORF for the A-subunit of the diphtheria exotoxin with an inserted stop-codon.

The GAPr was inserted after the subcloning of the two cDNAs obtained by RT-PCR (with heat-resistant polymerase Pfu II having a proofreading activity) on the genomic RNA matrix taken from a (+)ssRNA-containing virus (in the case of HCV, a patient's blood was the source of the genomic RNA). One of the cDNA was obtained from the first 2.8 kb of the genomic RNA, including the 5'-UTR and the frame encoding structural proteins. The other was taken from the 3'-UTR. The treatment of the amplification products and GAPr with the above restriction endonucleases leads to a possibility of their linkage in a reaction with T4-bacteriophage ligase. The GAPr® was cloned in reverse orientation relative to structural proteins ORF. The resulting constructs can be built into any of the following plasmids' polylinker: pCI® (Proega), pCMVB® (Invitrogene), and pcDNA3.1® (Clonetech).

DNA plasmids pCI::RNA_{HCV}::GAPr) were introduced into the cells of the HepG2 line with the help of the transformation with Transfectin® method (frequency 2,550-1,200 clones per 10⁵ cells). The transformed cells were selected in the medium RPMI 1640 with 10% fetal calf serum and geneticin (0.5 mg/ml). The (G418^{R}) geneticin-resistant clones were used to determine HCV-specific antigens, RNA SRNA_{HCV} and GAPr.

24-72 hours after the electroporation HCV-specific antigens and RNA, and also the diphtheria toxin, started to be detected in G418^{R} cells. It was shown that no morphological signs of death of the G418^{R} cells from the action of diphtheria toxin were noted either after 72 hour or longer (up to 14 days). In 14 days, HCV-like particles containing SRNA_{HCV} were detected in the G418^{R} cells culture medium. The human hepatocarcinoma cells HepG2 expressing NS-proteins (the HepG2NS line) turned out to be highly sensitive to the medium in which the G418^{R} cells were cultured, dissolved up to the titer of 1:1,600.

### Example 2

HCV-"antivirus" was used as antiviral RNA. Amplification was achieved via PCR with thermopolymerase Pwo II, 2 oligonucleotid primers and the plasmid pCI:: DNA matrix (SRNA^{HCV}::GAPr). The amplicon was used for its transcription by the T7 bacteriophage RNA-polymerase. The RNA obtained was inserted into the cells of the HepG2NS line by electroporation. 24 hours after electroporation the diphtheria toxin could be detected in the cells. The first diphtheria toxin was detected in the course of 48-52 hours after the electroporation and continued to be detected during 120 hours.

In 6 hours, HCV-specific antigens and SRNA_{HCV}, and the diphtheria toxin could be detected in the cells. The toxin itself could be detected by ELISA. Obvious morphological signs of cell destruction were detected 28 hours after electroporation. HCV-like particles containing SRN_{HCV} were detected in the HepG2NS-line porated cell culture medium in 14 days. The non-tracted HepG2Ns-line cells appeared to be highly sensitive to the medium in which porated cells had been cultured, dissolved up to the titer of 1:100.

## Claims

1. An anti-Hepatitis virus agent, comprising the following components (A) to (B),
(A) a nucleotide sequence directing the synthesis of a complementary strand of the hepatitis-virus;
(B) a nucleotide sequence containing at least a regulatory region operably linked to a structural gene encoding a toxin exhibiting a LD50 for vertebrates of not more than 100ng/kg body weight;
wherein the nucleotide sequence encoding the toxin is positioned in an antisense direction.

2. The anti-virus agent according to claim 1, wherein component (A) is derived from the 5'- and/or 3'-untranslated regions of a Hepatitis virus type B, C, D and or E.

3. The anti-virus agent according to claim 2, wherein the component(A) is derived from the 3' untranslated region of the HCV-virus.

4. The anti-virus agent according to any of the preceding claims, wherein the regulatory region comprises the Shine-Dalgarno sequence or the internal ribosomal binding site (IRBS) of the genomic RNA of the poliovirus vaccine strain Sabin 2.

5. The anti-virus agent according to any of the preceding claims, wherein the toxin is seleted from the group comprising diphteria exotoxin, diphtheria exotoxin A-subunit, Shigella toxin, Disenteria toxin.

6. The anti-virus agent according to any of the preceding claims, which comprises a DNA or a RNA vector.

7. Use of an anti-virus agent according to any of the preceding claims for the manufacture of a medicament for treating hepatitis.

8. The use according to claim 7, wherein the viral disease is caused by a hepatitis virus type B, C, D and or E.

## Patentansprüche

1. Anti-Hepatitisvirus-Agens, das die folgenden Bestandteile (A) bis (B) umfasst,
(A) eine Nukleotidsequenz, die die Synthese eines komplementären Stranges des Hepatitisvirus steuert;
(B) eine Nukleotidsequenz, die mindestens einen regulatorischen Bereich enthält, der mit einem Strukturgen funktionell verbunden ist, das ein Toxin kodiert, das fiir Vertebraten eine LD50 von nicht mehr als 100 ng/kg Körpergewicht aufweist.

2. Anti-Hepatitisvirus-Agens nach Anspruch 1, worin der Bestandteil (A) von den 5'und/oder 3'-nicht-translatierten Bereichen eines Hepatitisvirus des Typs B, C, D und/oder E abgeleitet ist.

3. Anti-Hepatitisvirus-Agens nach Anspruch 2, worin der Bestandteil (A) von dem 3'nicht-translatierten Bereich des HCV-Virus abgeleitet ist.

4. Anti-Hepatitisvirus-Agens nach einem der vorstehenden Ansprüche, worin der regulatorische Bereich die Shine-Dalgano-Sequenz oder die interne ribosomale Bindungsstelle (IRBS) der genomischen RNA des Impfstammes Sabin 2 des Poliovirus umfasst.

5. Anti-Hepatitisvirus-Agens nach einem der vorstehenden Ansprüche, worin das Toxin ausgewählt ist aus der Gruppe umfassend das Diphterieexotoxin, die A-Untereinheit des Diphterieexotoxins, das Shigellentoxin und das Dysenterietoxin.

6. Anti-Hepatitisvirus-Agens nach einem der vorstehenden Ansprüche, das einen DNAoder einen RNA-Vektor umfasst.

7. Verwendung eines anti-viralen Agens nach einem der vorstehenden Ansprüche zur Herstellung eines Medikaments zur Behandlung von Hepatitis.

8. Verwendung nach Anspruch 7, wobei die virale Erkrankung durch einen Hepatitisvirus des Typs B, C, D und/oder E verursacht wird.

## Revendications

1. Un agent anti-viral de l'hépatite comprenant les composants (A) à (B) suivants,
(A) une séquence de nucléotides dirigeant la synthèse d'un brin complémentaire du virus de l'hépatite;
(B) une séquence de nucléotides contenant au moins une région régulatrice liée opérationnellement à un gène structural codant une toxine ayant une LD50 pour les vertébrés pas supérieure à 100 ng/kg de poids corporel ;
dans lequel la séquence de nucléotides codant la toxine est positionnée dans une direction anti-sens.

2. L'agent anti-viral selon la revendication 1 dans lequel le composant (A) est dérivé des régions non translatées 5'- et/ou 3' d'un virus de l'hépatite de type B, C, D et/ou E.

3. L'agent anti-viral selon la revendication 2 dans lequel le composant (A) est dérivé de la région non translatée 3' du virus HCV.

4. L'agent anti-viral selon l'une quelconque des revendications précédentes dans lequel la région régulatrice comprend la séquence Shine-Dalgarno ou le site interne de liaison ribosomal (IRBS) de l'ARN génomique de la souche vaccinale Sabin 2 du virus de la poliomyélite.

5. L'agent anti-viral selon l'une quelconque des revendications précédentes dans lequel la toxine est choisie à partir du groupe comprenant l'exotoxine de la diphtérie, la sous-unité A de l'exotoxine de la diphtérie, la toxine de Shigella, la toxine de Disenteria.

6. L'agent anti-viral selon l'une quelconque des revendications précédentes comprenant un vecteur ADN ou ARN.

7. Utilisation d'un agent anti-viral selon l'une quelconque des revendications précédentes pour la fabrication d'un médicament pour le traitement de l'hépatite.

8. L'utilisation selon la revendication 7 dans laquelle la maladie virale est causée par le virus de l'hépatite de type B, C, D ou E.
